# EUROPEAN PATENT SPECIFICATION

(11) **EP 4 108 193 B1**
(45) Date of publication and mention of the grant of the patent: **29.11.2023**
(21) Application number: 21382556.5
(22) Date of filing: 23.06.2021
(51) Int. Cl.: A61B 17/80

(54) **RIB FRACTURE REPAIR SYSTEM**
REPARATURSYSTEM FÜR RIPPENBRUCH
SYSTÈME DE RÉPARATION DE FRACTURE DE CÔTES

(43) Date of publication of application: 28.12.2022
(73) Proprietor: Fundación de la Comunidad Valenciana Hospital General para la Investigación Biomédica, Docencia y Desarrollo de las Ciencias de la Salud, 46014 Valencia (ES)
(72) Inventor: ZARAGOZA FERNÁNDEZ, Cristóbal, 46014 Valencia (ES); GUIJARRO JORGE, Ricardo, 46014 Valencia (ES); LÓPEZ ALCINA, Emilio, 46014 Valencia (ES); GILABERT ESTELLES, Juan, 46014 Valencia (ES)
(74) Representative: ZBM Patents - Zea, Barlocci & Markvardsen

(56) References cited:
- US-A1- 2013 131 738
- US-A1- 2015 209 093
- US-A1- 2018 125 547
- US-A1- 2019 159 819

## Description

### Technical field

The present invention relates to rib fracture repair systems.

### BACKGROUND

Rib fractures are the most common type of trauma injuries. They are typically treated using rib fixation devices that are applied to a fractured rib. Known rib fixation devices make use of cables, clamps, metal plates and fixation screws that extend into the bone.

The fractures are conventionally repaired by screwing a plate onto the bone to hold it in place such that it heals properly. This conventional practice has however the disadvantage that incisions are required to be formed in the bone. Said incisions are formed in correspondence with holes provided in the plate for insertion of screws to secure the plate to the bone. This results in extensive trauma, pain, and time required for healing the fracture and tissue overlying the bone. A further disadvantage of this approach relates to possible incorrect placement of screws as a result of poor accuracy of bone incisions.

US2013131738A1 shows embodiments of a fixation clamp to be secured over an outer periphery of a fractured bone. The fixation clamp comprises clamp members including first and second arms, a locking screw, and a set screw having a threaded tip for engaging an outer surface of the first arm. The first arm of the clamp member is inserted into a channel of the second arm until the clamp member contacts an outer surface of the bone. The set screw of the clamp member is screwed into the second arm until a threaded tip thereof engages the first arm such that the first and second clamp members are held provisionally in position over the bone. The locking screw is then driven into channels drawing the first and second clamp assemblies toward one another and applying a radially compressive force to the bone.

Although later prior art approaches have been found to be advantageous since no bone incisions are required in the bone, there is still a need for more efficient fixation devices in terms of bone clamping for an effective rib fracture repair which at the same time are simple and cost effective.

### SUMMARY

A rib fracture repair system is disclosed herein with which the above disadvantages are at least partially overcome and through which additional advantages are achieved.

The present rib fracture repair system comprises at least two clamp assemblies that may be arranged spaced a certain distance apart from each other. The separation distance of the clamp assemblies can be adjusted.

A shaped plate is slidably held between both clamp assemblies. A contact surface may be defined in the shaped plate to contact the rib when the rib fracture repair system is applied thereto.

The rib has a curved outer periphery. At least a first and a second rib transverse dimensions are defined in the rib. Rib transverse dimensions are defined herein as dimensions associated with the cross-section of the rib.

Each clamp assembly includes an upper clamp and a lower clamp.

At least one of the upper clamp, the lower clamp and the shaped plate may be preferably made of a medical grade titanium alloy such as Ti6Al4V. In general, at least one of the upper clamp, the lower clamp and the shaped plate is preferred to be made of a material suitable for withstanding stresses without breaking and avoiding permanent deformation.

In use, the upper and lower clamps may at least partially surround the outer periphery of the rib, as stated above, for holding the rib together with the shaped plate. For this purpose, at least one of the upper clamp and the lower clamp may preferably have a curved surface to fit said curved outer periphery of the rib.

The upper clamp and the lower clamp are attached to each other through a connecting member. The connecting member is preferably configured for rotatably attaching the upper clamp and the lower clamp to each other. The fact that the upper clamp and the lower clamp can be rotated with respect to each other results in the rib fracture repair system to be effectively adapted to a wide range of different rib morphologies.

The clamp assemblies are configured such that they can be adjusted according to a first direction, a second direction, and a third direction. Said first, second, and a third directions are orthogonal to each other. The clamp assemblies are therefore allowed to be adjusted according to three orthogonal directions. As a result, the rib fracture repair system can be effectively adapted to a wide range of different rib morphologies. Advantageously, the clamp assemblies may precisely accommodate the curved outer periphery of the rib at any point thereof.

The first direction according to which the clamp assemblies can be adjusted is a direction along which the clamp assemblies can be moved along the length of the shaped plate. It may be preferred that the shaped plate is guided in the lower clamp so that the clamp assemblies can be moved in said first direction. For this purpose, a guiding portion may be formed in the lower clamp for guidance of the shaped plate.

The second direction according to which the clamp assemblies can be adjusted is a direction along which the upper clamp can be moved towards and away from the contact surface of the shaped plate so as to adapt to the first rib transverse dimension.

The third direction according to which the clamp assemblies can be adjusted is a direction along which the upper clamp can be moved towards and away from the lower clamp so as to adapt to the second rib transverse dimension.

Fixing means may be provided to fix the clamp assemblies in said first, second, and third directions. The fixing means allow to hold the clamp assemblies in position once the rib fracture repair system has been applied. For this purpose, the fixing means may be suitable to fix the clamp assemblies from being moved along one or more of the above mentioned first, second, and third directions.

The fixing means may for example comprise at least a first screw to hold the shaped plate to the lower clamp. The first screw may be a grub screw or any suitable threaded bolt suitable for fastening the lower clamp and the shaped plate to each other. The shaped plate can be thus fixed in position by the grub screw pressing against an inner wall of the above mentioned guiding portion formed in the lower clamp for guidance of the shaped plate.

The fixing means may also comprise a second screw to hold the upper clamp to the lower clamp. The connecting member may be adapted for receiving said second screw to hold the upper clamp to the lower clamp in an adjustable manner.

An adjusting screw may be also provided to adjust the upper clamp along the second direction according to the first rib transverse dimension.

In one example, at least one portion of the lower clamp may be spaced apart a distance away from the rib, in use. This may prevent veins, arteries and nerves running along the underside of the rib from being compressed, avoiding damages. In another example, said distance between at least one portion of the lower clamp and rib may not be necessary so that at least one portion of the lower clamp may abut the rib in use.

The upper clamp or the lower clamp, or both the upper clamp and the lower clamp, may have contact surfaces intended to contact the rib. In one example, the contact surfaces may be any suitable frictional contact surface such as, for example, a surface with indentations. Frictional contact surfaces in at least one of the upper and lower clamps provide enhanced grip of the rib fracture repair system to the outer periphery of the rib.

With the present rib fracture repair system, repair of costal fractures due to polytrauma is highly improved. Lower invasiveness is achieved since the use of screw connections to the bone structure is avoided. Thus, greater safety is provided since the risk of damaging intercostal vessels and nerves is reduced.

With the present rib fracture repair system, dissection of the inside of the rib to place the rib fracture repair system is not required. In addition, there are no components that can be lost inside the body during placement or the implantation period.

The present rib fracture repair system has been shown to be highly versatile. It is adaptable to any type of rib trauma. Furthermore, minimal instrumentation is required for rib fixation and easy removal since it can be easily removed once the fracture has adequately consolidated.

### BRIEF DESCRIPTION OF THE DRAWINGS

Non-limiting examples of the present rib fracture repair system will be described in the following, with reference to the appended drawings.

In the drawings:
Figure 1 is a general perspective view of a first example of the rib fracture repair system;
Figure 2 is a cross-sectional view of the rib fracture repair system in figure 1; and
Figure 3 is a cross-sectional view of a second example of the rib fracture repair system.

### DETAILED DESCRIPTION OF EXAMPLES

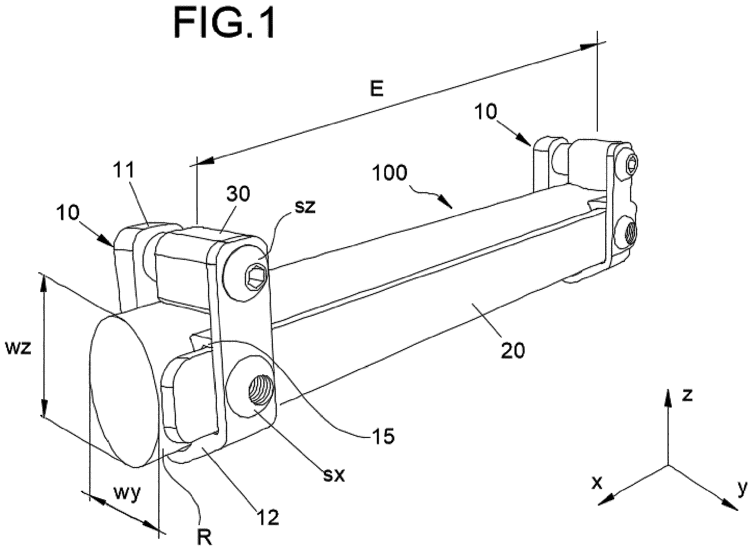

Two examples of the rib fracture repair system 100 are disclosed and shown in the figures 1-3 of the drawings.

In both examples, the rib fracture repair system 100 comprises two clamp assemblies 10. The clamp assemblies 10 are spaced by an adjustable distance E apart from each other as shown in figure 1.

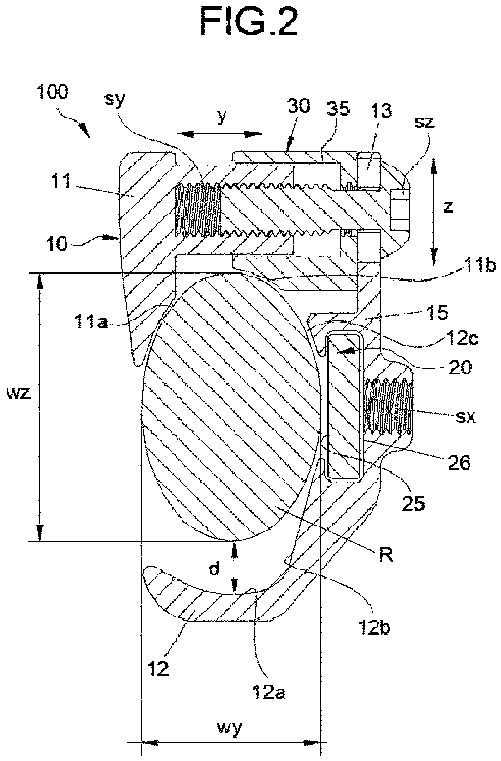

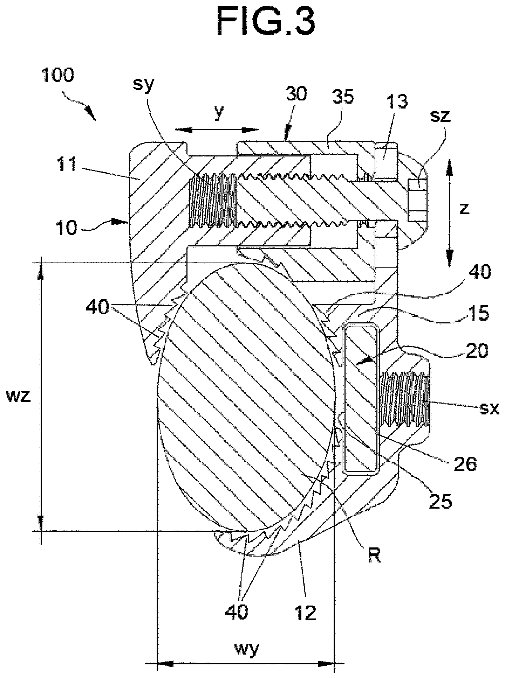

Between both clamp assemblies 10 a shaped plate 20 is slidably held. As shown in figures 2 and 3 of the drawings, a contact surface 25 is defined in the shaped plate 20 to contact a curved outer periphery a rib R when the rib fracture repair system 100 is applied.

The clamp assemblies 10 each include an upper clamp 11 and a lower clamp 12. The upper clamp 11 and the lower clamp 12 together with the shaped plate 20 are configured for holding the rib R, as shown in the figures.

The upper and lower clamps 11, 12 and the shaped plate 20 are made of a medical grade titanium alloy such as Ti6Al4V or any suitable material for withstanding stresses without breaking and avoiding permanent deformation.

Clamps 11, 12 are configured to have curved contact surfaces adapt to the curved outer periphery of the rib R. Thus, in use, the outer periphery of the rib R is at least partially surrounded by the upper and lower clamps 11, 12 as shown in the figures of the drawings.

In the first example shown in figures 1 and 2 of the drawings, the contact surfaces 11a, 11b, 11c of the upper clamp 11 and the contact surfaces 12a, 12b of the lower clamp 12 are smooth surfaces.

In the second example shown in figure 3, the contact surfaces of the upper clamp 11 11a, 11b, 11c and the contact surfaces 12; 12a, 12b of the lower clamp are frictional surfaces including indentations 40. The indentations 40 are a formed as recesses or notches on the surface of the contact surfaces 11a, 11b, 11c, 12; 12a, 12b of the upper and lower clamps 11, 12.

The frictional contact surfaces 11a, 11b, 11c, 12; 12a, 12b in the second example shown in figure 3 provide enhanced grip of the rib fracture repair system 100 to the outer periphery of the rib R.

Figures 2 and 3 are cross-sectional views of first and second examples of the rib fracture repair system 100 applied to a rib R. As shown in said figures 2, 3, the rib R has a curved outer periphery and a cross-sectional configuration defined by first transverse dimension wy and a second transverse dimension wz.

The clamp assemblies 10 are configured such that they can be adjusted according to a first direction x. The first direction x is a direction along which the clamp assemblies 10 can be moved along the length of the shaped plate 20. The shaped plate 20 is guided along a guiding portion 15 formed in the lower clamp 12 such that the clamp assemblies 10 can be moved along the first direction x to adjust positioning of clamp assemblies 10 as well as the separation distance E there between.

The clamp assemblies 10 are also configured such that they can be adjusted according to a second direction y, that is, a horizontal direction in figures 2 and 3 of the drawings. The second direction y is a direction along which the upper clamp 11 can be moved towards and away from the contact surface 25 of the shaped plate 20 so as to adapt to the above mentioned first rib transverse dimension wy. Adjustment along the second direction y allows the clamp assemblies 10 to be adjusted within a range of between about -2,5 mm and about +2,5 mm.

The clamp assemblies 10 are also configured such that they can be adjusted according to a third direction z, that is, a vertical direction in figures 2 and 3 of the drawings. The third direction z is a direction along which the upper clamp 11 can be moved towards and away from the lower clamp 12 so as to adapt to the above mentioned second rib transverse dimension wz. Adjustment along the third direction z allows the clamp assemblies 10 to be adjusted within a range of between about -1,25 mm and about +1,25 mm.

As shown in the figures, the first, second, and third directions x, y, z are orthogonal to each other.

The clamp assemblies 10 can be fixed in position in said first, second, and third directions x, y, z. For this purpose, a grub screw sx is provided. The grub screw sx is threaded into the lower clamp 12 such that as it is screwed into the upper clamp 11 a free end of the grub screw sx abuts against a tightening surface 26 of the shaped plate 20, opposite the contact surface 25 of the shaped plate 20. As a result, the shaped plate 20 is held in position to the lower clamp 12 preventing it from being moved along the first direction x.

A connecting member 30 is provided between the upper clamp 11 and the lower clamp 12. The connecting member 30 is configured for rotatably attaching the upper clamp 11 to the lower clamp 12 in an adjustable manner.

The connecting member 30 consists of an adjusting cylinder 35 configured for receiving therein an adjusting screw sy. The adjusting screw sy is arranged passing through a groove 13 that is formed in one upper end of the lower clamp 12. One free end of the adjusting screw sy is threaded into an internal thread formed in the upper clamp 11. Rotation of the adjusting screw sy results in displacement of the upper clamp 11 along the second direction y. This allows the upper clamp 11 to be moved towards and away from the lower clamp 12 such that positioning of the upper clamp 11 can be accurately adjusted with respect to the first rib transverse dimension wy.

A further screw sz is also provided passing through the groove 13 in the lower clamp 12. The screw sz allows the upper clamp 11 to be held to the lower clamp 12 in an adjustable manner along the third direction z.

Screws sx, sy, sz allow the clamp assemblies 10 to be effectively and accurately adapted to the shape of the rib R at any point thereof. No connections to the rib R involving screws and holes are required.

In general, contact surfaces 12a, 12b of the lower clamp 12 and contact surfaces 11a, 11b of the upper clamp 11 are arranged to contact the rib R. However, in the example shown in figure 2, the lower surface 12a of the lower clamp 12 is spaced apart away from the rib R by a distance d, so such lower surface 12a does not come into contact with the rib R when the rib fracture repair system 100 is applied. Distance d is suitable for preventing veins, arteries and nerves running along the underside of the rib from being compressed, avoiding damages.

In the example shown in figure 3, the contact surfaces 12a, 12b, 12c of the lower clamp 12a and the contact surfaces 11a, 11b of the upper clamp 11 are arranged to contact the outer periphery of the rib R. In this example, contact surfaces 11a, 11b, 12a, 12b, 12c are frictional contact surfaces and include indentations 40. Frictional contact surfaces 11a, 11b, 12a, 12b, 12c allow for a better grip of upper and lower clamps 11, 12 on the curved outer periphery of the rib R.

## Claims

1. Rib fracture repair system (100) comprising at least two clamp assemblies (10) between which a shaped plate (20) is slidably held, each clamp assembly (10) including an upper clamp (11) and a lower clamp (12) attached to each other through a connecting member (30), the upper and lower clamps (11, 12) being shaped for holding a rib (R) together with the shaped plate (20), wherein the clamp assemblies (10) are configured such that they can be adjusted according to:
- a first direction (x) along which the clamp assemblies (10) can be moved along the length of the shaped plate (20);
- a second direction (y) along which the upper clamp (11) can be moved towards and away from a contact surface (25) of the shaped plate (20) so as to adapt to a first rib transverse dimension (wy); and
- a third direction (z) along which the upper clamp (11) can be moved towards and away from the lower clamp (12) so as to adapt to a second rib transverse dimension (wz); and
wherein the first, second, and third directions (x, y, z) are orthogonal to each other.

2. The rib fracture repair system (100) of claim 1, wherein the upper clamp (11) and the lower clamp (12) are rotatably attached to each other.

3. The rib fracture repair system (100) of claim 1 or 2, wherein the shaped plate (20) is guided in the lower clamp (12) so that the clamp assemblies (10) can be moved in the first direction (x).

4. The rib fracture repair system (100) of any of the preceding claims, wherein it includes fixing means (sx, sz) to fix the clamp assemblies (10) in one or more of the first, second, and third directions (x, y, z).

5. The rib fracture repair system (100) of claim 4, wherein the fixing means comprise at least a first screw (sx) to hold the shaped plate (20) to the lower clamp (12) and a second screw (sz) to hold the upper clamp (11) to the lower clamp (12).

6. The rib fracture repair system (100) of claim 5, wherein the first screw (sx) is a grub screw.

7. The rib fracture repair system (100) of any of the preceding claims, wherein it includes an adjusting screw (sy) to adjust the upper clamp (11) along the second direction (y) according to first rib transverse dimension (wy).

8. The rib fracture repair system (100) of any of the claims 5-7, wherein the connecting member (30) is adapted for receiving the second screw (sz) to hold the upper clamp (11) to the lower clamp (12) in an adjustable manner.

9. The rib fracture repair system (100) of any of the preceding claims, wherein the upper and lower clamps (11, 12) are shaped to at least partially surround the outer periphery of the rib (R).

10. The rib fracture repair system (100) of any of the preceding claims, wherein at least one of the upper clamp (11) and the lower clamp (12) have contact surfaces (11a, 11b, 11c; 12a, 12b) intended to contact the rib (R).

11. The rib fracture repair system (100) of claim 10, wherein at least one of the contact surfaces (11a, 11b, 11c; 12a, 12b) includes indentations (40).

12. The rib fracture repair system (100) of any of the preceding claims, wherein at least one of the upper clamp (11) and the lower clamps (12) has a curved surface to fit a outer periphery of the rib (R).

13. The rib fracture repair system (100) of any of the preceding claims, wherein, in use, at least one portion of the lower clamp (12) is spaced apart a distance (d) away from the rib (R).

14. The rib fracture repair system (100) of any of the preceding claims, wherein at least one of the upper clamp (11), the lower clamp (12), and the shaped plate (20) is made of a medical grade titanium alloy.

15. The rib fracture repair system (100) of claim 14, wherein said medical grade titanium alloy is Ti6Al4V.

## Patentansprüche

1. Rippenbruchreparatursystem (100) umfassend mindestens zwei Klemmenbaugruppen (10) zwischen denen eine geformte Platte (20) verschiebbar gehalten ist, wobei jede Klemmenbaugruppe (10) eine obere Klemme (11) und eine untere Klemme (12) umfasst, die über ein Verbindungselement (30) aneinander befestigt sind, wobei die obere und die untere Klemme (11, 12) zum Halten einer Rippe (R) zusammen mit der geformten Platte (20) geformt sind, wobei die Klemmenbaugruppen (10) so konfiguriert sind, dass sie eingestellt werden können gemäß:
- einer ersten Richtung (x), entlang der die Klemmenbaugruppen (10) entlang der Länge der geformten Platte (20) bewegt werden können;
- einer zweiten Richtung (y), entlang der die obere Klemme (11) zu einer Kontaktfläche (25) der geformten Platte (20) hin und von dieser weg bewegt werden kann, um sich an eine erste Querabmessung (wy) der Rippe anzupassen; und
- einer dritten Richtung (z), entlang der die obere Klemme (11) zu der unteren Klemme (12) hin und von dieser weg bewegt werden kann, um sich an eine zweite Querabmessung (wz) der Rippe anzupassen; und
wobei die erste, zweite und dritte Richtung (x, y, z) orthogonal zueinander sind.

2. Das Rippenbruchreparatursystem (100) von Anspruch 1, wobei die obere Klemme (11) und die untere Klemme (12) drehbar aneinander befestigt sind.

3. Das Rippenbruchreparatursystem (100) von Anspruch 1 oder 2, wobei die geformte Platte (20) in der unteren Klemme (12) geführt ist, so dass die Klemmenbaugruppen (10) in der ersten Richtung (x) bewegt werden können.

4. Das Rippenbruchreparatursystem (100) von einem der vorhergehenden Ansprüche, wobei es Befestigungsmittel (sx, sz) beinhaltet, um die Klemmenbaugruppen (10) in einer oder mehreren von der ersten, zweiten und dritten Richtung (x, y, z) zu befestigen.

5. Das Rippenbruchreparatursystem (100) von Anspruch 4, wobei das Befestigungsmittel mindestens eine erste Schraube (sx), um die geformte Platte (20) an der unteren Klemme (12) zu halten, und eine zweite Schraube (sz), um die obere Klemme (11) an der unteren Klemme (12) zu halten, umfasst.

6. Das Rippenbruchreparatursystem (100) von Anspruch 5, wobei die erste Schraube (sx) eine Regulierschraube ist.

7. Das Rippenbruchreparatursystem (100) von einem der vorhergehenden Ansprüche, wobei es eine Einstellschraube (sy) beinhaltet, um die obere Klemme (11) entlang der zweiten Richtung (y) gemäß der ersten Querabmessung (wy) der Rippe einzustellen.

8. Das Rippenbruchreparatursystem (100) von einem der Ansprüche 5 bis 7, wobei das Verbindungselement (30) angepasst ist, um die zweite Schraube (sz) aufzunehmen, um die obere Klemme (11) an der unteren Klemme (12) in einer einstellbaren Weise zu halten.

9. Das Rippenbruchreparatursystem (100) von einem der vorhergehenden Ansprüche, wobei die obere und untere Klemme (11, 12) so geformt sind, dass sie den Außenumfang der Rippe (R) zumindest teilweise umgeben.

10. Das Rippenbruchreparatursystem (100) von einem der vorhergehenden Ansprüche, wobei mindestens eine von der oberen Klemme (11) und der unteren Klemme (12) Kontaktflächen (11a, 11b, 11c; 12a, 12b) aufweist, die dazu bestimmt sind, die Rippe (R) zu berühren.

11. Das Rippenbruchreparatursystem (100) von Anspruch 10, wobei mindestens eine der Kontaktflächen (11a, 11b, 11c; 12a, 12b) Vertiefungen (40) beinhaltet.

12. Das Rippenbruchreparatursystem (100) von einem der vorhergehenden Ansprüche, wobei mindestens eine von der oberen Klemme (11) und der unteren Klemmen (12) eine gekrümmte Oberfläche aufweist, um zu einem Außenumfang der Rippe (R) zu passen.

13. Das Rippenbruchreparatursystem (100) von einem der vorhergehenden Ansprüche, wobei bei der Verwendung mindestens ein Abschnitt der unteren Klemme (12) um einen Abstand (d) von der Rippe (R) beabstandet ist.

14. Das Rippenbruchreparatursystem (100) von einem der vorhergehenden Ansprüche, wobei mindestens eine von der oberen Klemme (11), der unteren Klemme (12) und der geformten Platte (20) aus einer Titanlegierung von medizinischer Qualität hergestellt ist.

15. Das Rippenbruchreparatursystem (100) von Anspruch 14, wobei die Titanlegierung von medizinischer Qualität Ti6Al4V ist.

## Revendications

1. Système de réparation de fracture de côte (100) comprenant au moins deux ensembles de serrage (10) entre lesquels une plaque façonnée (20) est maintenue de manière coulissante, chaque ensemble de serrage (10) comprenant un élément de serrage supérieur (11) et un élément de serrage inférieur (12) fixés l'un à l'autre par un élément de connexion (30), les éléments de serrage supérieure et inférieure (11, 12) étant formées pour maintenir une côte (R) avec la plaque façonnée (20), dans lequel les ensembles de serrage (10) sont configurés de telle sorte qu'ils peuvent être ajustés selon :
- une première direction (x) le long de laquelle les ensembles de serrage (10) peuvent être déplacés le long de la longueur de la plaque façonnée (20) ;
- une deuxième direction (y) le long de laquelle l'élément de serrage supérieur (11) peut être rapproché et éloigné d'une surface de contact (25) de la plaque façonnée (20) de manière à s'adapter à une première dimension transversale de côte (wy) ; et
- une troisième direction (z) le long de laquelle l'élément de serrage supérieur (11) peut être rapproché et éloigné de l'élément de serrage inférieure (12) de manière à s'adapter à une deuxième dimension transversale de côte (wz) ; et
dans lequel les première, deuxième et troisième directions (x, y, z) sont orthogonales les unes aux autres.

2. Le système de réparation de fracture de côte (100) de la revendication 1, dans lequel l'élément de serrage supérieur (11) et l'élément de serrage inférieur (12) sont fixés de manière rotative l'un à l'autre.

3. Le système de réparation de fracture de côte (100) de la revendication 1 ou 2, dans lequel la plaque façonnée (20) est guidée dans l'élément de serrage inférieure (12) de sorte que les ensembles de serrage (10) peuvent être déplacés dans la première direction (x).

4. Le système de réparation de fracture de côte (100) de l'une quelconque des revendications précédentes, dans lequel il comprend un moyen de fixation (sx, sz) pour fixer les ensembles de serrage (10) dans une ou plusieurs des première, deuxième et troisième directions (x, y, z).

5. Le système de réparation de fracture de côte (100) de la revendication 4, dans lequel le moyen de fixation comprend au moins une première vis (sx) pour maintenir la plaque façonnée (20) sur l'élément de serrage inférieur (12) et une seconde vis (sz) pour maintenir l'élément de serrage supérieur (11) sur l'élément de serrage inférieur (12).

6. Le système de réparation de fracture de côte (100) de la revendication 5, dans lequel la première vis (sx) est une vis sans tête.

7. Le système de réparation de fracture de côte (100) de l'une quelconque des revendications précédentes, dans lequel il comprend une vis de réglage (sy) pour régler l'élément de serrage supérieur (11) le long de la seconde direction (y) en fonction de la première dimension transversale de côte (wy).

8. Le système de réparation de fracture de côte (100) de l'une quelconque des revendications 5 à 7, dans lequel l'élément de connexion (30) est adapté pour recevoir la seconde vis (sz) pour maintenir l'élément de serrage supérieur (11) contre l'élément de serrage inférieur (12) d'une manière réglable.

9. Le système de réparation de fracture de côte (100) de l'une quelconque des revendications précédentes, dans lequel les éléments de serrage supérieur et inférieur (11, 12) sont formés de manière qu'ils entourent au moins en partie la périphérie extérieure de la côte (R).

10. Le système de réparation de fracture de côte (100) de l'une quelconque des revendications précédentes, dans lequel au moins l'un de l'élément de serrage supérieur (11) et de l'élément de serrage inférieur (12) ont des surfaces de contact (11a, 11b, 11c; 12a, 12b) destinées à entrer en contact avec la côte (R).

11. Le système de réparation de fracture de côte (100) de la revendication 10, dans lequel au moins l'une des surfaces de contact (11a, 11b, 11c ; 12a, 12b) comprend des indentations (40).

12. Le système de réparation de fracture de côte (100) de l'une quelconque des revendications précédentes, dans lequel au moins l'un de l'élément de serrage supérieur (11) et des éléments de serrage inférieurs (12) a une surface incurvée de manière à s'adapter à une périphérie extérieure de la côte (R).

13. Le système de réparation de fracture de côte (100) de l'une quelconque des revendications précédentes, dans lequel, lors de son emploi, au moins une partie de l'élément de serrage inférieur (12) est espacée d'une distance (d) de la côte (R).

14. Le système de réparation de fracture de côte (100) de l'une quelconque des revendications précédentes, dans lequel au moins l'un de l'élément de serrage supérieur (11), de l'élément de serrage inférieur (12) et de la plaque façonnée (20) est fait(e) en un alliage de titane de qualité médicale.

15. Le système de réparation de fracture de côte (100) de la revendication 14, dans lequel ledit alliage de titane de qualité médicale est Ti6Al4V.
